(19) Europäisches Patentamt
European Patent Office
Office européen des brevets

(11) Publication number: 0 335 624 A2

# (12) EUROPEAN PATENT APPLICATION

(21) Application number: **89302981.9**

(22) Date of filing: **23.03.89**

(51) Int. Cl.[4]: **C 08 F 246/00**
C 08 F 220/54, A 61 K 7/48,
C 02 F 11/12

(30) Priority: **28.03.88 US 174082**

(43) Date of publication of application:
**04.10.89 Bulletin 89/40**

(84) Designated Contracting States:
**AT BE CH DE ES FR GB GR IT LI LU NL SE**

(71) Applicant: **Macrochem Corporation**
**21X Olympia Avenue**
**Woburn Massachusetts 01801 (US)**

(72) Inventor: **Samour, Carlos M.**
**324 Linden Street**
**Wellesley Massachusetts 02181 (US)**

**Krauser, Scott, F.**
**19 Gage Street**
**Methuen Massachusetts 01844 (US)**

(74) Representative: **Adams, William Gordon et al**
**RAWORTH, MOSS & COOK 36 Sydenham Road**
**Croydon Surrey CR0 2EF (GB)**

## (54) Surface active polymers.

(57) Water-soluble or water-swellable polymers ionic in character and having surface-active properties are provided which are based on a lipophilic monomer, which may be an ionic, nonionic and anionic water-soluble monomer, the latter monomer being further characterized by its homopolymer being soluble in water at a pH in the range of 2 to 14. Other monomers may be present. It is preferred that these be water-soluble. The polymers are prepared generally in the usual manner employed for α,β-ethylenically unsaturated compounds. The products have a myriad of uses, such as thickeners, water-treating aids, sewage treatment, superabsorbers, etc.

EP 0 335 624 A2

## Description

### Surface-Active Polymers

The present invention relates to new polymers which are surface active and which carry high positive (cationic) charges, negative (anionic) charges, or both (ampholytic) charges. The polymers are water-soluble or water-swellable products whose properties can be varied widely and tailored for innumerable applications by variations in the charge densities and by the different monomers and their ratios. More particularly, the polymers of the present invention are interpolymers of two or more polymerizable monomers, at least one being a lipophilic group containing anionic, cationic, ampholytic, or nonionic monomers and at least one being a water-soluble ionic monomer which may be anionic, cationic, or ampholytic. Water-soluble nonionic monomers may be present.

### Background of the Invention and Prior Art

Water soluble polymers derived from water-soluble monomers are, of course, well known. Polyvinyl pyrrolidone, polyacrylic acid and salts thereof, polyvinyl methyl ether, polyacrylamide, poly(2-hydroxyethyl) acrylate, are illustrative of such polymers. Copolymers, terpolymers, interpolymers, graft copolymers, polymer "alloy" compositions, etc. of the monomeric procursors of the above mentioned polymers with another water-soluble monomer(s) or water-insoluble monomers such as alkyl acrylates, alkyl methacrylates, vinyl acetate, vinyl propionate, styrene, among, literally, thousands of such monomers are also known. See, for example U.S. Patents 4,057,623; 4,057,625; and 4,098,987. Surface-active polymerizable compounds of the anionic, cationic, and ampholytic and nonionic types can be found in U.S. Patents 3,718,693; 3,759,982; 3,839,419; 3,925,4422; 3,928,423; 3,936,492; 4,011,259; 3,983,166; and 4,029,658. These patents, in the main, describe the compounds as useful emulsion stabilizers in the emulsion polymerization of insoluble monomers and particularly monomers which produce water-insoluble end products. The stabilizers are polymerizable monomers which become an integral unit in an interpolymer.

Still other anionic polymers can be found in U.S. Patent 3,066,032; Japanese 78/10682; European Patent Application 29,970; and U.S. Patent 4,284,517. Other nonionic polymers can be found in U.S. Patent 4,278,277; 4,352,916; and 4,384,974. Cationic polymers are also described in U.S. Patents 2,883,370; 2,923,701; 3,288,770; 3,551,384; 3,554,318; and 3,689,468. See also U.S. Patents 4,075,183 and 4,138,446.

Further reference is made to U.S. Patent 4,415,717 which describes certain copolymers of a surface-active quaternary ammonium salt monomer and from 50 wt% to 97 wt% of acrylamide. The patentor finds it surprising that the molecular weight of the polymeric cationic surfactants "can be substantially greater than 10,000 and still remain water soluble and surface active" (Col. 2, Lines 31 to 36).

The polymers of the present invention are water soluble or water-swellable and may be of low, medium, high or very high molecular weight in contrast to those of the prior art and particularly those in U.S. Patent 4,415,717. The polymers of the present invention have higher charge densities than those of U.S. Patent 4,415,717 and in this regard alone are far superior for many uses where improved or enhanced utility is related to the polymer charge.

### Detailed Description of the Invention

The polymers of the present invention are based on a mixture of polymerizable monomers which comprise on the one hand a lipophilic monomer which may be anionic, cationic, nonionic, or ampholytic, and on the other hand an ionic, water-soluble monomer. There may also be present other ionic or nonionic monomers which are also preferred to be characterized as water-soluble.

The monomer make-up of the compositions of this invention is as follows:

A) from 0.5% to about 60% by weight of a polymerizable ionic or nonionic lipophilic group containing monomer;

B) from about 40% to 99.5% by weight of a polymerizable water-soluble, ionic monomer, which is also soluble in homopolymeric form in water at a pH range of 2 to 14; and

C) from 0 to about 60% by weight of polymerizable monomer, preferably nonionic and water-soluble and different from monomers (A) and (B).

The lipophile-containing monomer is preferably a polymerizable ethylenically unsaturated monomer containing an average of $C_8$ to $C_{30}$ and preferably $C_8$ to $C_{24}$ and more preferably $C_9$ to $C_{18}$ group which confers a lipophilic character to the compound and polymers derived therefrom. The $C_8$ to $C_{30}$ may be aliphatic or aromatic and preferably is alkyl or substituted alkyl in the aliphatic series and alkylphenyl in the aromatic series. Particularly preferred lipophilic moieties include dodecyl, dodecenyl, tetradecyl, hexadecyl, linoleyl, eicosyl, n-octylphenyl, n-nonylphenyl, dodecylphenyl, and the like.

As pointed out above, the lipophilic monomer (A) may be anionic, cationic, nonionic, or ampholytic. General formulas of particularly useful lipophiles are as follows:

I. Acrylic and Methacrylic Cationic Monomers

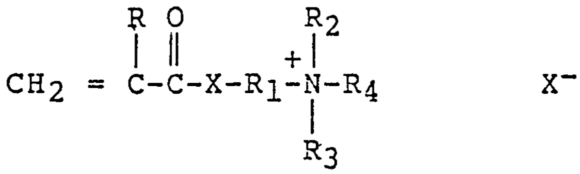

wherein R is H or $CH_3$-; X is -O- or $-\overset{R}{N}-$; $R_1$ is alkylene group, $C_2$ to $C_8$, preferably ethylene and propylene; $R_2$ and $R_3$ low alkyl groups ($C_1$ to $C_4$) preferably methyl; $R_1$, a lipophilic group havin 8-30 carbon atoms, preferably $C_{12}$ to $C_{24}$. The lipophilic group is an alkyl, alkenyl, or alkylaryl group. The radical $X^-$ is a halogen atom, preferably a chloride ion, or an alkyl sulfate, such as methyl sulfate.

II. Allylic and Diallylic Cationic Monomers

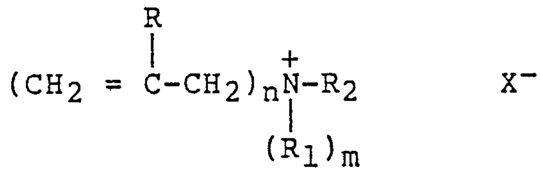

when n is 1, m is 2, and when n is 2, m is 1; R is H or $CH_3$; $R_1$ is low alkyl groups ($C_1$ to $C_4$), preferably methyl; $R_2$ is a lipophilic group as defined under Formula I for $R_4$; $X^-$ as defined above under Formula I.

III. Acrylic and Methacrylic Nonionic Monomers

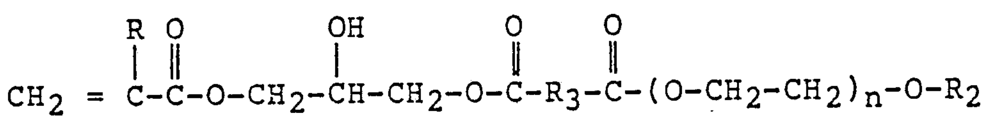

wherein R is H or $CH_3$-; $R_3$ is ethylene, propylene or O-phthalyl; n is 5 - 50, preferably 5 to 35; $R_2$ a lipophilic group as defined in Formula I for $R_4$.

IV. Maleate (Fumarate) Anionic/Nonionic Monomers

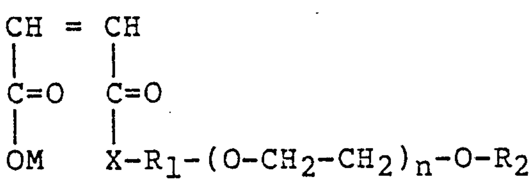

wherein M, X, $R_1$, n and $R_2$ are as defined under Formula III above.

V. Vinyl Cationic Monomers

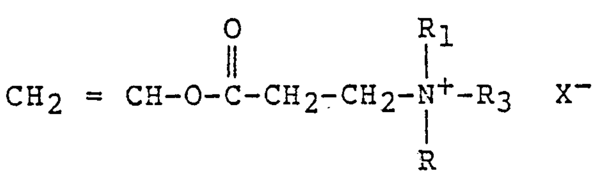

wherein R and $R_1$ are lower alkyl groups ($C_1$ to $C_4$) preferably methyl; $R_3$ is a lipophilic group as defined in Formula I for $R_4$; $X^-$ as defined in Formula I for $X^-$.

VI. Acrylic (Methacrylic) and Allylic Nonionic Monomers

$V-W-CH_2-CH_2-(OCH_2-CH_2)_n-OR_2$

when V is

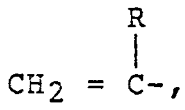 , W is 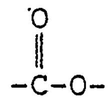 or 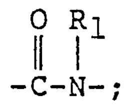 ; R is H or $CH_3$

R is H or $CH_3$ and $R_1$ is lower alkyl group ($C_1$ to $C_4$), preferably methyl. When V is allyl or methallyl, W is $-OCH_2-CH_2-$; n = 5 to 50, preferably 5 to 35 and $R_2$ is a lipophilic group as defined in Formula I for $R_4$.

Some specific compounds within the foregoing Formulas I through VI are:

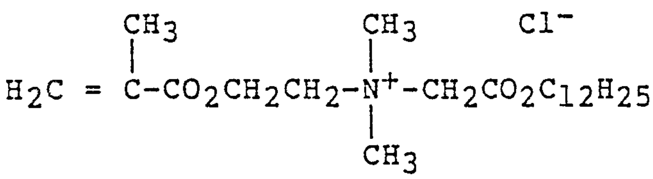

COO1

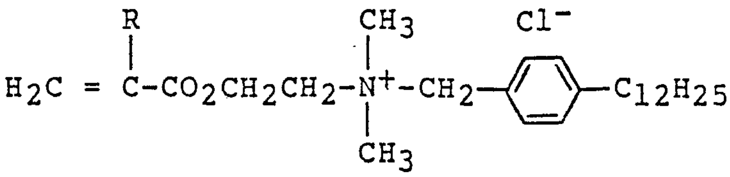

CO02 (R=CH3)
CO10 (R=H)

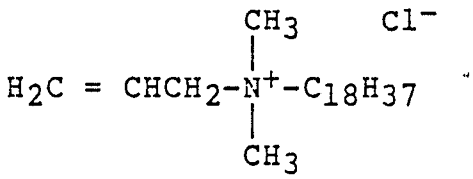

CO03

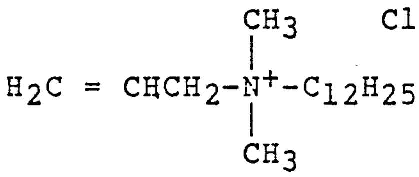

CO04

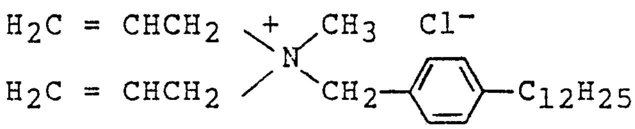

CO05

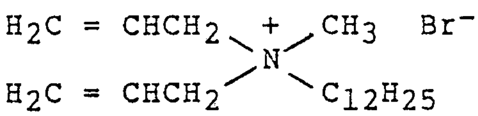

CO06

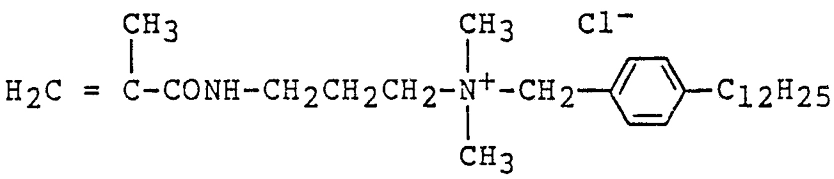

CO07

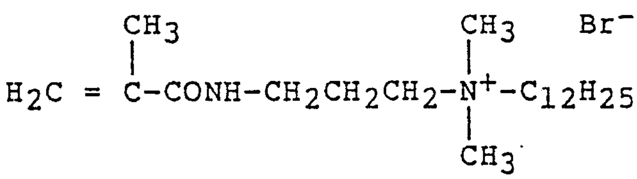

CO08

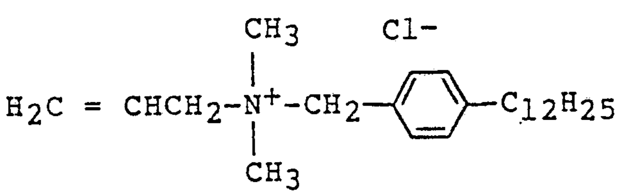

CO09

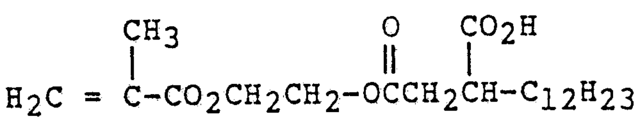

A002

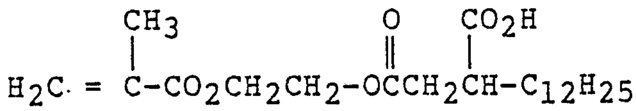

A003

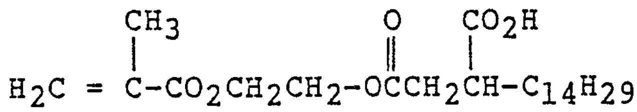

A004

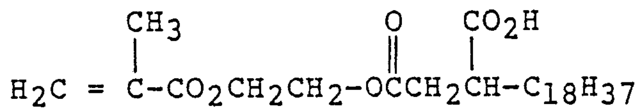

A005

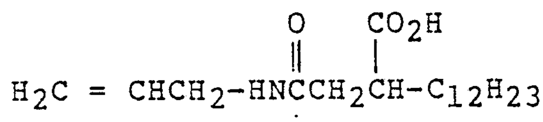

A006

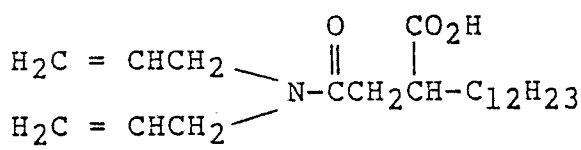

A007

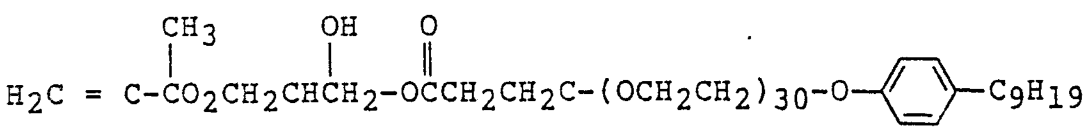

N001

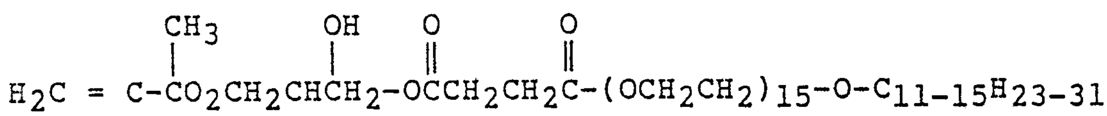

NOO2

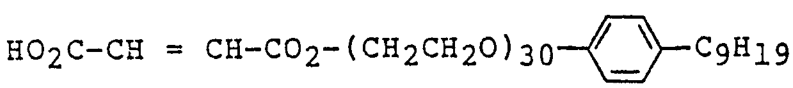

NOO3

The above monomers are given by way of illustration only, as coming within the foregoing Formulas I to VI. It is, of course, understood that many other lipophilic monomers may be used in this invention. Reference is made to U.S. Patents cited below with descriptions and the disclosure of these patents in their entirety is hereby incorporated by reference thereto.

U.S. Patent No. 3,714,256 - Describe diallyl and dimethallyl alkyl lipophilic benzyl ammonium halides.

U.S. Patent No. 3,718,693 - Describe quaternary ammonium salts having an N-diallylacetamido or N-dimethallylacetamido radical.

U.S. Patent No. 3,751,451 - Describe quaternary ammonium monomers having a lipophilic group covalently linked to the quaternary nitrogen through hydroxysuccinyloxy or hydroxysuccinylamino radicals.

U.S. Patent No. 3,759,982 - Describe anionic complexes of quaternary ammonium monomers.

U.S. Patent No. 3,780,092 - Describe quaternary monomers containing a lipophilic radical.

U.S. Patent No. 3,839,419 - Similar to U.S. 3,751,451.

U.S. Patent No. 3,879,447 - Describe quaternary ammonium salt monomers having diallyl or dimethyallyl and a lipophilic radical covalently linked to the quaternized nitrogen.
U.S. Patent No. 3,898,274 - Similar to U.S. 3,751,451.
U.S. Patent No. 3,925,442 - Describe a complex of quaternary ammonium salt monomer and an anionic surfactant.
U.S. Patent No. 3,928,423 - Describe maleic and itaconic quaternary ammonium salt monomers containing a lipophilic radical attached to the quaternary nitrogen.
U.S. Patent No. 3,936,492 - Describe quaternary ammonium salt monomers containing a lipophilic group joined to the quaternary nitrogen atom.
U.S. Patent No. 3,959,355 - Describe allyl, methallyl, and vinyl acetate esters of a quaternary ammonium monomer and containing an alkyl succinoxy radical.
U.S. Patent No. 3,960,935 - Describe monomers containing a lipophilic radical, a polyethyleneoxy group and an ethylenically unsaturated radical.
U.S. Patent No. 3,983,166 - Describe a complex of a quaternary ammonium salt monomer and an anionic surfactant.
U.S. Patent No. 4,011,259 - Describe quaternary ammonium salt monomers containing acid amido groups derived from maleic, citraconic and itaconic acids.
U.S. Patent No. 4,029,658 - Describe quaternary ammonium salt monomer complex with an anionic surfactant. The monomers contain allyl and a methallyl radical.
U.S. Patent No. 4,051,158 - Describe a quaternary ammonium salt monomer containing an α,β-unsaturated polymerizable group and a hydroxysuccinylamino or a hydroxysuccinyloxy radical.
U.S. Patent No. 4,064,091 - Describe ethylenically-unsaturated monomer containing quaternary nitrogen.
U.S. Patent No. 4,198,520 - Describe monomers containing a lipophilic radical, a polyethyleneoxy group and an ethylenically unsaturated radical.

The water-soluble ionic monomer (B) may be any of the well-known water-soluble monomers which are also water-soluble in homopolymeric form within the pH range of 2 to 14. The preferred pH range in the use of these polymers is 4 to 10. Illustrative monomers are acrylic acid, methacrylic acid, and monomers similar to Formulas I to VI but having no lipophilic group.

Illustrative monomers of the cationic type include:

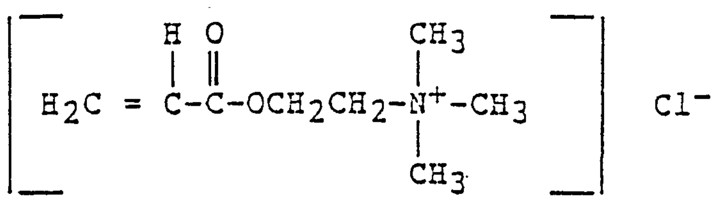

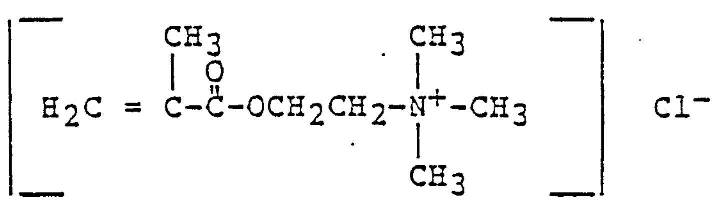

Typical anionics include:

$H_2C = CH{-}COO^-M^+$ (M is e.g. Na)

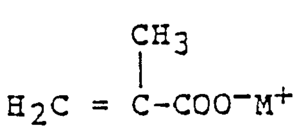 (M is e.g. Na)

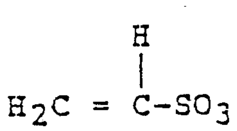 (M is e.g. Na)

As described above, a third comonomer (or more) may be present up to 60% by weight. These can be of any type, ionic or nonionic, ampholytic, but should preferably be water-soluble in the monomeric state.

Examples of comonomer (C) include; acrylamide, methacrylamide, N-vinyl pyrrolidone, N-vinyl oxazolidinone, vinyl methyl ether, hydroxyethyl acrylate, hydroxyethyl methacrylate, allyl alcohol, allyl methyl ether, N,N-dimethylaminoethyl methacrylate and its hydrochloride salt, etc.

The general procedures for carrying out the polymerizations of the monomer combinations of this invention are varied and illustrative general techniques are given below.

## Polymerization Procedures

Method A - Azo Initiation

The monomer mix, solvent and initiator are placed in a reaction kettle and purged with $N_2$ for about 1/2 hour. The flask is surrounded by a water bath at the required polymerization temperature and the mixture heated and stirred under $N_2$ for approximately 6 hours to complete the reaction.

**Example:**

A 1 L kettle containing 180 g (75% aq., 0.652 mol) of (N-trimethylammonium ethyl methacrylate chloride) 21.4 g (HLM COO2) (70% in isopropanol, 0.0332 mol), 550 g of $H_2O$ and 0.050 g (0.155 mmol) of 2,2′-azobis(N,N′-dimethylene-isobutyramidine) dihydrochloride (VA-044) is purged with $N_2$ for 1/2 hour and then heated by a water bath at 50° C. The mixture thickens after 1.5 hours and is rubbery after 5 hours. After 6 hours the product is removed and found to be essentially soluble in $H_2O$. Brookfield viscosities (12 rpm, #3) of 1% and 0.1% solutions are 5900 and 870 cps, respectively.

Method B - Redox Initiation

The monomer mix and solvent in a reaction kettle are purged with $N_2$ for approximately 1/2 hour with stirring and then equal weights of ammonium persulfate and sodium metabisulfite added. Polymerizations are usually evident within minutes and accompanied by exotherms of 0-50° C. Products are removed within 1 hour.

**Example:**

A 500 ml resin kettle containing 202.5 g (50% aq.) of acrylic acid, 11.25 g of methacrylatoethyl dodecenylsuccinate (A002), 78.75 g of 1,2-dimethoxyethane, and 62.5 g of $H_2O$ are purged with $N_2$ for 1/2 hour. To the kettle is added 0.165 g (0.723 mmol) of ammonium persulfate and 0.166 g (0.873 mmol) of sodium metabisulfite, each in 10 ml of $H_2O$. Within 3 minutes the temperature has risen to 80°C and the mixture is highly viscous. The product is cooled gradually over about 1 hour and removed. It shows no significant thickening of water before or after basification to pH 8 with $NH_4OH$.

Method C - Batch polymer with ethylenediaminetetraacetic acid (EDTA)

Procedure:

The acrylamide monomer mix, water, initiator, and 250-750 ppm EDTA are sparged with $N_2$ for at least 30 min. with stirring. A water bath at the desired reaction temperature is then applied and maintained. Polymerization is soon evident and within 60 min., usually less than 30, stirring is stopped since the viscosity is too high for useful mixing. The internal temperature normally rises and within 20 more minutes peaks at about 10° C above that of the bath. The polymerization is completed by further heating for 4 1/2 - 5 1/2 hours.

Example:

To a 500 ml reaction kettle is added 98.04 g of 50% aq. acrylamide, 1.43 g of 70% methacrylatoethyldimethyl(dodecylbenzyl)ammonium chloride (C002) in i-PrOH, 400.6 g $H_2O$, and 0.0250 g of EDTA. The resulting mixture is heated by a 50° C water bath while being stirred and vigorously sparged with $N_2$. After 40 min., with the internal temperature at 50°C, 0.55 ml of 0.50M 2,2′-azobis(2-amidinopropane) dihydrochloride (V-50) is added via syringe. Within 7 minutes the mixture has become highly viscous and stirring is stopped. After 0.5 hours more the internal temperature has increased to 64° C, followed by gradual cooling. Heating at 50° C is maintained an additional 5 hours to give the product as a somewhat hazy gel.

Method D - Batch polymerization with mercaptan

Procedure:

A solution of monomers and mercapto compound in water is stirred and degassed with $N_2$ and to it is added the initiator. Within 10 min. the temperature has begun to rise to eventual exotherms of 11-65°C, following which the mixtures are allowed to cool gradually to room temperature.

**Example:**

A kettle containing 108 g (1.50 mol) acrylic acid, 12.0 g (0.0300 mol) A002, 1.41 g (0.0153 mol) mercaptoacetic acid, and 180 g of $H_2O$ at 27° C is sparged with $N_2$ for 45 min. and to it added 0.495 g (0.00153

mol) VA-044. The temperature quickly begins to rise and this continues until this main exotherm subsides, the cooling bath is removed, and the mixture allowed to come to ambient temperature over several hours of continued stirring. The final product solution has a viscosity of 1,000 cps.

Method E - Delayed Addition of Ingredients

Procedure 1.

A resin kettle is equipped for the separate additions of (1) the monomer mix, (2) the initiator, and (3) the mercapto compound, the latter two as water solutions. The reaction solvent, water and/or isopropanol, is placed in the kettle and heated to the desired polymerization temperature (60 to 80°C) while sparging with $N_2$. Ten percent of the initiator solution is added and then simultaneous addition of the three components begun. The rates are adjusted such that the monomers and mercapto compound are added over approximately 90 min. and the remaining initiator over about 120 min. Following this the reaction temperature is maintained for at least 60 min. more.

**Example:**

One hundred and ten grams of water in a 500 ml reaction kettle is heated under $N_2$ to 80°C over 45 min. To this is added 0.407 g (0.00150 mol) of V-50 in 5.0 ml of $H_2O$, and separate dropwise additions of (1) 108 g (1.50 mol) of acrylic acid and 17.14 g (12.0 g active, 0.0265 mol) of 70% C002 in isopropanol, (2) 1.4 g (0.013 mol) of thioglycerol in 30 ml $H_2O$, and (3) 3.563 g (0.0131 mol) of V-50 in 30 ml of $H_2O$ are begun. The rates are such that these additions require 87, 92, and 100 minutes, respectively. The temperature is then maintained at 82 to 83°C for 85 minutes. On cooling, the resulting polymer solution has a viscosity of 2500 cps.

Procedure 2.

As in Procedure 1, except that the initial kettle charge contains 10% of each of the reaction components, and addition of remaining monomer/mercapto and initiator is over a time span of about 45 and 75 minutes, respectively.

Procedure 3.

As in Procedure 2, except that the initial kettle charge contains 50% of each of the reaction components.

Procedure 4.

As in Procedure 1, except that the monomers are added as a water/isopropanol solution and the mercaptan is in isopropanol.

Many of the lipophilic monomers of the cationic type of Formula I and the anionic type of Formula III are known and their preparation evident among the prior art cited earlier. As an illustration, the cationic monomer identified above as C002 may be readily prepared as follows:
A charge of 78.5 g (0.5 moles) of dimethylaminoethyl methacrylate (Sipomer 2M1M from Alcolac) and 147.5 g (0.5 moles) of dodecylbenzyl chloride are stirred together at room temperature for half an hour, and then left at room temperature for 24 hours. The yield of 226.0 g of a glassy material can be dissolved in 675 ml of water to make a 25% aqueous solution.
The cationic C004 can be made as follows:
A mixture of 85.7 g (0.4 moles) of lauryldimethylamine and 30.6 g (0.4 moles) of allyl chloride are stirred for one hour at room temperature. After standing overnight at room temperature, 116 g of white crystallized N,N-dimethyl-N-allyl-N-laurylammonium chloride is obtained.
The diallyl cationic C005 may be synthesized as follows:
In 122 g of chloroform are mixed 88.5 g (0.3 moles) of dodecylbenzyl chloride and 33.3 g (0.3 moles) of N-methyldiallylamine (Virginia Chemical Co.). About 6 mg of hydroquinone monomethyl ether is added to the solution and the mixture is refluxed for 6 hours at 70°C after which the chloroform is removed under vacuum. The yield is 122 g of N-methyl-N,N-diallyl-N-dodecylbenzylammonium chloride which can be made up as a 50% aqueous solution.
The foregoing synthesis may also be carried out in the absence of the chloroform solvent.
The cationic monomer C007, N,N-dimethyl-N-dodecylbenzyl-N-propylenemethacrylamide ammonium chloride, is prepared by stirring together equimolar amounts of dimethylaminopropylmethacrylamide and dodecylbenzyl chloride in isopropanol at room temperature for 30 minutes. The solution becomes viscous and the temperature is raised to 40°C (from 25°C). Stirring is stopped and the solution is kept at room temperature. The anionic monomer A002 is prepared by reacting 26 g (0.2 moles) of hydroxyethyl methacrylate and 53.2 g (0.2 moles ) dodecenyl succinic anhydride in the presence of 3.0 g of potassium acetate in 150 ml of ethyl acetate with stirring at 60°C for 16 hours. The solution is cooled to room temperature, filtered, and the ethyl acetate removed under vacuum to yield 79.2 g of a viscous liquid which is the monomer A002. Monomer N003 is made by the equimolar reaction of maleic anhydride (10.1 g, 0.103 moles) and nonylphenoxy polyethyleneoxy ethanol (3O E.O. groups) (158 g, O.1O3 moles)--available as Igepal CO-88O from GAF Corporation--with stirring at 8O°C for 8 hours under anhydrous conditions. The yield of product, which is a waxy solid, is 168 g.

Many of the nonionic lipophilic monomers are also old as, again, can be seen from the prior art described

above. Of particular significance and novel is a class of lipophilic monomers of the formula.

VII

$$CH_2 = \overset{\overset{R}{|}}{C}-C-X-R_1-(OCH_2-CH_2)_n-O-\overset{\overset{O}{\|}}{C}-CH_2-CH-\underset{\underset{R_2}{|}}{C}OO^- \quad M^+$$

wherein R is hydrogen or methyl; X is -O- or $-\overset{R}{\overset{|}{N}}-$; $R_1$ is alkylene, preferably $C_2$ or $C_3$; n is O to 5O and preferably O to 35; $R_2$ is defined for $R_2$ in Formula III and $M^+$ is hydrogen, ammonium, sodium ion, organic amine, etc.

Compounds of Formula VII may be prepared by reacting the $R_2$- substituted succinic compound (e.g. dodecenyl succinic anhydride) with the hydroxy-substituted acrylic ester or amide (e.g. hydroxyethyl methacrylate or hydroxyethyl methacrylamide) or the ethoxylated (1 to 5O moles of ethylene oxide) derivatives thereof. An illustrative preparation of

$$CH_2 = \overset{\overset{CH_3}{|}}{C}-CONHCH_2CH_2\ \underset{\underset{O}{\|}}{O C}-CH_2\ \underset{\underset{C_{12}H_{23}}{|}}{C}HCOOH$$

(dodecenyl)

involves heating, with stirring, a mixture of O.2 moles of hydroxyethylmethacrylamide, O.2 moles dodecenyl succinic anhydride and 3.O g. potassium acetate in 15O ml ethylacetate for 16 hours at 6O°C. Cool to room temperature, filter and remove the ethyl acetate at reduced temperature and under vacuum to constant weight of 76 g. By repeating the foregoing procedure with (separately each time) 1E.O, 2E.O, 1OE.O, 2OE.O, 3OE.O and 5OE.O condensate of hydroxyethyl methacrylamide, the corresponding ethyoxylated derivative of Formula VII where n is 1, 2, 5, 1O, 2O, 3O and 5O, respectively are obtained.

The interpolymers of this invention have a variety of uses which are a function, among other factors, of their charge strength and distribution, surface activity, flexibility in the hydrophilic-lipophilic balance and molecular weight.

Polymeric surfactants are also called "polysoaps," and their uses encompass thickening functions, uses as flocculants, antistats, anti-bacterial agents, adhesion- promoting agents, coatings, binders, protective colloids, dry strength additives for paper, textile sizings, textile soil release agents, anti-redeposition agents in detergents, filtering aids, cleansers, electrostatic coatings, superabsorbers for water (as in disposable diapers), water-soluble or water-sensitive, pH-responsive films for packaging detergents, in printing inks for rheology control, and the like.

The following examples will serve to illustrate the present invention without being deemed limitative thereof. Parts are by weight unless otherwise indicated.

**Example 1.**

Into a suitable reaction vessel equipped with thermometer, stirrer, inert gas inlet and outlet tubes, there is introduced 9 parts of N,N,N-trimethyl ethyl methacrylate ammonium chloride (monomer A) and 1 part of N,N-dimethyl-N-dodecylbenzyl ethyl methacrylate ammonium chloride (monomer B) and 90 parts of water. The solution is adjusted to a temperature of 25°C. Then 0.02 parts of ammonium persulfate is added followed by 0.01 parts of sodium metabisulfite. The polymerization is conducted under a blanket of nitrogen. The polymerization initiates soon after the catalyst addition and an exotherm of 20°C is observed over a period of 45 minutes. A viscous polymer solution is obtained. The Brookfield viscosity of 1% solution is 80 cps @ 12 RPM, using a No. 2 spindle.

**Example 2-6:**

Example 1 is repeated except different compositions of monomer A and monomer B (N,N-dimethyl-N-dodecylbenzyl ethyl methacrylate ammonium chloride) are employed as shown in Table I; percent solids is varied and Example 6 also contains 20 parts acrylamide in the monomer mix. Comparable results are achieved.

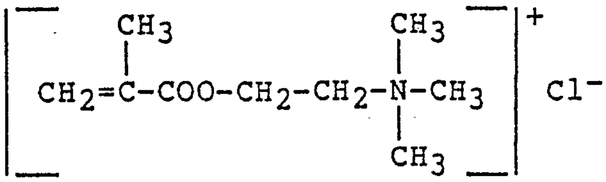

**Monomer A**

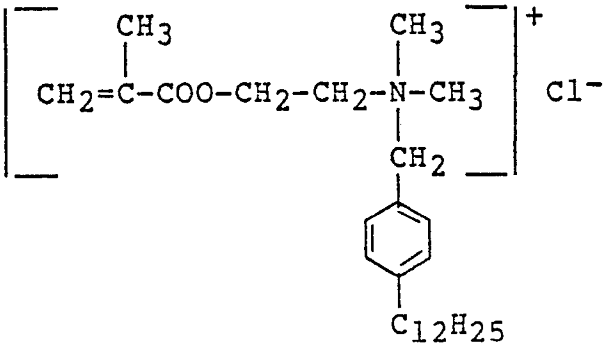

**Monomer B**

Table I

| Example: | Weight Ratio A:B | % Solids | of 1% Solution, cps @ 12 RPM |
|---|---|---|---|
| 2 | 85:15 | 20 | 6550 |
| 3 | 95:5 | 30 | 8700 |
| 4 | 4:1 | 5 | 23 |
| 5 | 9:1 | 20 | 5900 |
| 6 | 7:1:2 | 30 | 268 |

**Example 7-31:**

Example 1 is again repeated with the exception that different comonomers are used in varying proportions as shown in Table II.

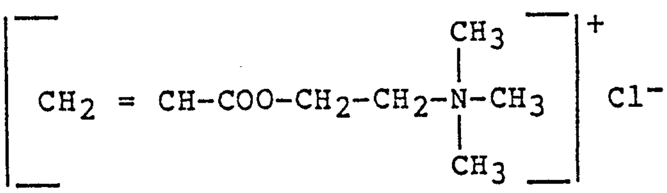

**Monomer $A_1$**

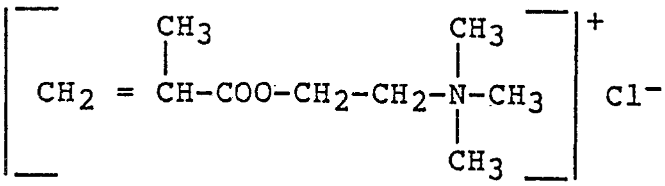

**Monomer $A_2$**

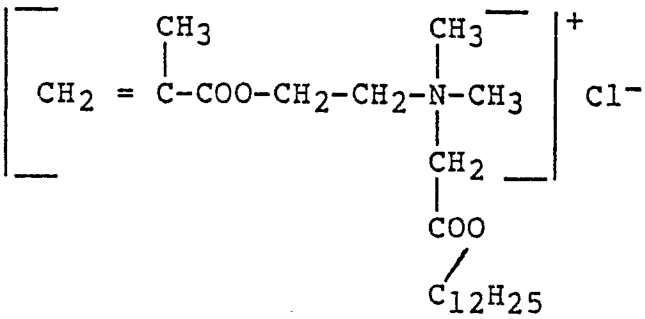

**Monomer $B_1$**

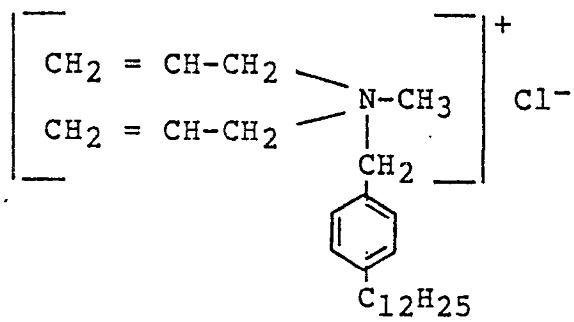

**Monomer $B_2$**

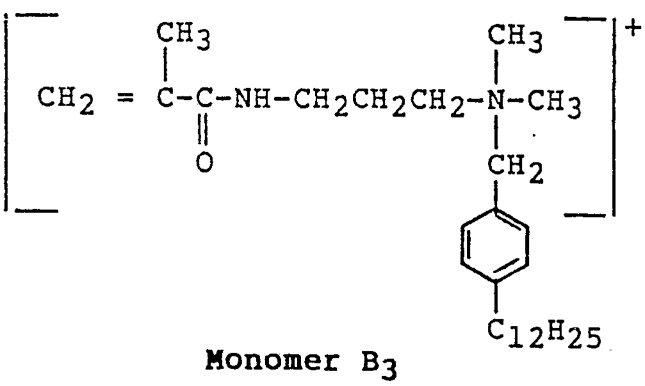

**Monomer $B_3$**

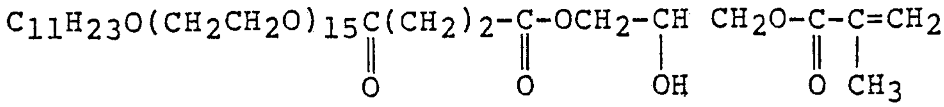

Monomer $B_4$

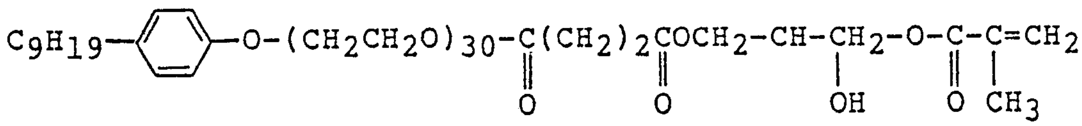

Monomer $B_5$

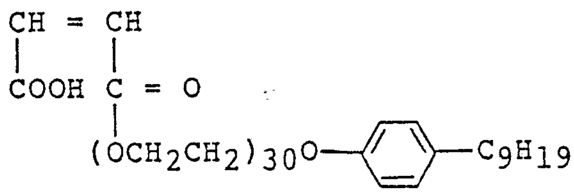

Monomer $B_6$

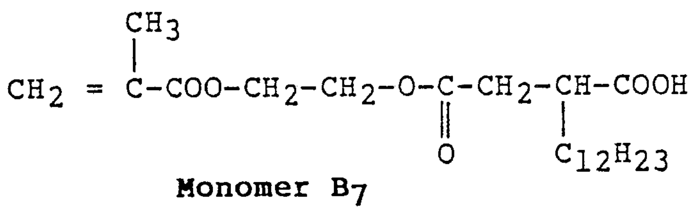

Monomer $B_7$

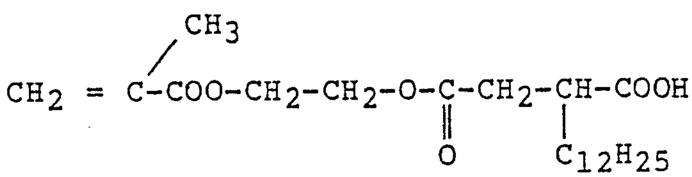

Monomer $B_8$

Table II

| Monomer A | Comonomer B | Example: | Weight Ratio |
|---|---|---|---|
| $A_1$ | $B_1$ | 7 | 7:1 |
| $A_2$ | $B_2$ | 8 | 90:10 |
| | | 9 | 95:5 |
| | | 10 | 98:2 |
| $A_2$ | $B_3$ | 11 | 80:20 |
| | | 12 | 90:10 |
| | | 13 | 95:5 |

Table II (cont.)

| Monomer A | Comonomer B | Example: | Weight Ratio |
|---|---|---|---|
| $CH_2$ = CH-COOH | $B_4$ | 14 | 80:20 |
| | | 15 | 90:10 |
| | | 16 | 95:5 |
| | | 17 | 98:2 |
| | | 18 | 99:1 |
| $CH_2$ = CH-COOH | $B_5$ | 19 | 80:20 |
| | | 20 | 90:10 |
| | | 21 | 95:5 |
| $CH_2$ = CH-COOH | $B_6$ | 22 | 75:25 |
| | | 23 | 80:20 |
| | | 24 | 90:10 |
| $CH_2$ = CH-COOH | $B_7$ | 25 | 80:20 |
| | | 26 | 90:10 |
| $CH_2$ = CH-COOH | $B_8$ | 27 | 80:20* |
| | | 28 | 90:10* |
| | | 29 | 95:5* |
| $CH_2$ = $CH-COO_2$/AAM | $B_7$ | 29a | 70:20:10** |

*Hard Gel.
**8000 viscosity

**Example 30:**

Examples 1 to 13 are each separately repeated except that in place of Monomer A, there is used an equal weight 1:1 mixture of Monomer A and acrylic acid.

**Example 31:**

Each of the foregoing examples 1 to 30 is separately repeated replacing one-half of Monomer A with each of the following:

1) N-vinyl pyrrolidone
2) Vinyl methyl ether
3) Acrylamide
4) Methacrylamide

Some classes of preferred polymers include from about 2% to 10% of monomer(s) A; from about 60 to 98% of monomer(s) B, and from about 0 to 30% of monomer(s) C. Other preferred ranges include from about 5% to about 30% monomer(s) A; 70 to 95% monomer B, and from about 0 to 25% monomer(s) C; still other preferred ranges cover about 2 to about 40% monomer(s) A, about 30% to about 40% monomer(s) B, and 30% to about 49% monomer(s) C.

Where mixtures of different monomer(s) A are used, such mixture may comprise 2 or 3 or more comonomers with each comprising from about .5% to about 99%. This is similarly so for monomers B and C as well, of noted preference and utility mention may be made of mixtures of monomer(s) B of different ionic character. For example, a 1:1 wt mixture of acrylic acid and a water-soluble cationic such as monomer A or $A_1$. All percents are given here by weight.

**Examples of Uses of Polymers of this Invention**

Example 1

A surface-active copolymer prepared from 90% AA and 10% A002 was used as the sole emulsifier in emulsion polymerization. A mixture of 2-ethylhexyl acrylate (45% of wt.), water (53.5%), and the interpolymer (1.5%), adjusted to pH 6 with ammonia, was stirred to give a stable pre-emulsion, which on heating in the presence of ammonium persulfate gave an emulsion polymer containing low levels of coagulum. A dried and cured film of this product had high tack and was useful as a pressure sensitive adhesive.

Example 2

The surface-active copolymer of the preceding example can be used as emulsifier and stabilizer in various cosmetic formulations, such as the following:

| | Wt.% |
|---|---|
| Water | 79.3 |
| Mineral Oil | 10 |
| Silicone Oil | 10 |
| Copolymer | 1 |
| Methyl Paraben | 0.5 |
| Triethanolamine | 0.2 |

The resulting preparation has a desirable feel and consistency, and moisturizes and protects when applied to the skin. Cleansing creams, after shave lotions, sunscreens, and hand or body lotions can be similarly prepared.

Example 3

The copolymers can also be used as thickeners and stabilizers in cut clears for textile pigment printing:

| | |
|---|---|
| Varsol 18 (aliphatic hydrocarbon) | 25% by wt. |
| Water | 25 |
| Sorbitan monoeolate | 4 |
| Sorbitan monooleate-E.O.20 | 8 |
| Sodium lauryl sulfate | 3 |
| Copolymer | 20 |
| 28% ammonia | 15 |

Example 4

The surface-active copolymers, especially those with higher charge densities, are useful in water treatment. For example, a product of copolymerization of dimethyladiallylammonium chloride (90% by wt.) and HLM-C005 (methyldiallyl(dodecylbenzyl)ammonium chloride, 10% by weight) in low concentrations will effectively flocculate particulate matter from raw water.

**Claims**

1. A water-soluble or water-swellable polymer derived from the polymerization of a mixture comprising

(A) from about 0.5% to about 50% by weight of a polymerizable, monomeric compound selected from the group consisting of anionic, cationic, nonionic and ampholytic,α, β-ethylenically unsaturated compounds and mixtures thereof, said monomeric compound containing at least one $C_8$ to $C_{30}$ lipophilic group selected from alkyl, alkenyl, and alkylaryl groups, and

(B) from about 99% to about 40% by weight of a monomeric, polymerizable, water-soluble non-lipophilic group containing at least one compound selected from the group consisting of anionic, cationic, and ampholytic α, β-ethylenically unsaturated compounds, which compound forms water-soluble homopolymers in the pH range of 2 to 14, and

(C) from 0 to about 60% by weight of at least one ionic or non-ionic monomer differing from monomers (A) and (B).

2. A water-soluble or water-swellable polymer as defined in Claim 1 wherein the lipophilic monomer is a cationic acrylic compound.

3. A polymer as defined in Claim 2 wherein the water-soluble monomer (B) is a cationic acrylic compound.

4. A polymer as defined in Claim 2 wherein the water-soluble monomber (B) is an anionic acrylic

compound.

5. A polymer as defined in Claim 2 wherein the water-soluble monomer (B) is an ampholytic compound.

6. A polymer as defined in Claim 1 wherein the lipophilic monomer is an anionic acrylic compound.

7. A polymer as defined in Claim 1 wherein the lipophilic monomer is a nonionic acrylic compound.

8. A polymer as defined in Claim 6 wherein the water-soluble monomer (B) is an anionic acrylic monomer.

9. A polymer as defined in Claim 7 wherein monomer (B) is an anionic acrylic monomer.

10. A polymer as defined in any one of the preceding claims wherein at least 1% of nonionic acrylic monomer (C) is present.

11. A polymer as defined in Claim 10 wherein monomer (C) is selected from the group consisting of acrylamide, N-methyl acrylamide, N-vinyl pyrrolidone, N-vinyl oxazolidinone, vinyl methyl ether, and vinyl alcohol.

12. A polymer as defined in Claim 2 wherein the lipophilic monomer has one of the following formulae:

I

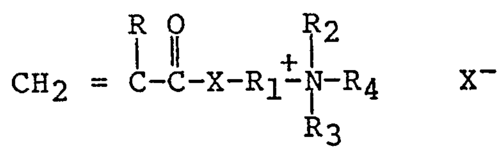

wherein R is H or $CH_3$-; X is -O- or

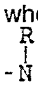

; $R_1$ is alkylene group, $C_2$ to $C_8$, preferably ethylene and propylene; $R_2$ and $R_3$, low alkyl groups ($C_1$ to $C_4$) preferably methyl; $R_1$, a lipophilic group having 8-30 carbon atoms, preferably $C_{12}$ to $C_{24}$, and wherein the lipophilic group is an alkyl, alkenyl, or alkylaryl group, the radical $X^-$ is a halogen atom, preferably a chloride ion, or an alkyl sulfate, such as methyl sulfate;

II

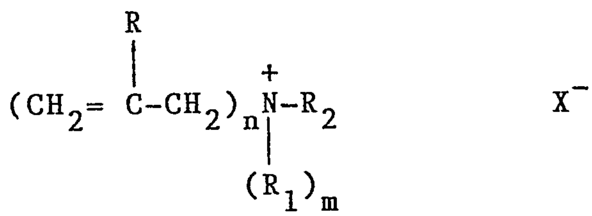

when n is 1, m is 2, and when n is 2, m is 1; R is H or $CH_3$; $R_1$ is low alkyl groups ($C_1$ to $C_4$), preferably methyl; $R_2$ is a lipophilic group as defined under Formula I for $R_4$; $X^-$ as defined above under Formula I; or

V

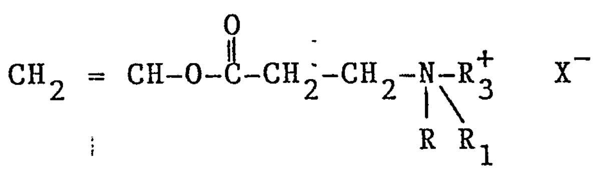

wherein R and $R_1$ are lower alkyl groups ($C_1$ to $C_4$) preferably methyl; $R_3$ is a lipophilic group as defined in Formula I for $R_4$; $X^-$ as defined in Formula I for $X^-$.

13. A polymer as defined in Claim 6 wherein the lipophilic monomer has the formula:

II

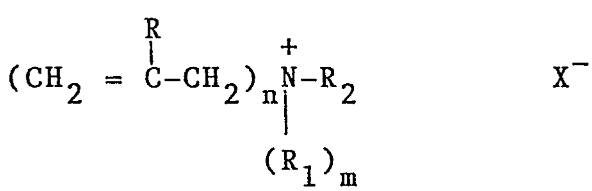

when n is 1, m is 2, and when n is 2, m is 1; R is H or $CH_3$; $R_1$ is low alkyl groups ($C_1$ to $C_4$), preferably methyl; $R_2$ is a lipophilic group as defined under Formula I for $R_4$; $X^-$ as defined above under Formula I.

14. A polymer as defined in Claim 7 wherein the lipophilic monomer has one of the following formulae:

V

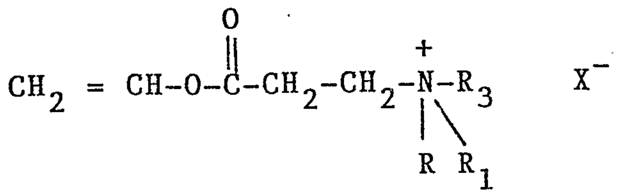

wherein R and $R_1$ are lower alkyl groups ($C_1$ to $C_4$) preferably methyl; $R_3$ is a lipophilic group as defined in Formula I for $R_4$; $X^-$ as defined in Formula I for $X^-$; or

VI V-W-$(OCH_2-CH_2)_n$-$OR_2$

when V is

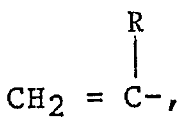 W is 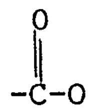 or 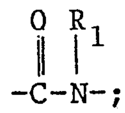

R is H or $CH_3$ and $R_1$ is lower alkyl group ($C_1$ to $C_4$ ), preferably methyl, wherein V is allyl or methallyl, W is $-OCH_2-CH_2-$; n = 5 to 50, preferably 5 to 35 and $R_2$ is a lipophilic group as defined in Formula I for $R_4$.

15. A process for preparing a polymer as defined in any one of the preceding claims which comprises interpolymerizing the monomers in the presence of a free radical catalyst at a temperature above about 0°C to no more than about 250°C for a sufficient time to effect substantially complete polymer formulation.

16. A process as defined in Claim 15 wherein the catalyst is a peroxygen compound.

17. A process as defined in Claim 15 wherein the process is carried out batchwise.

18. In a method for the purification of water and/or dewatering aqueous slurries, the improvement which comprises utilizing a polymer as defined in any one of Claims 1 to 14.

19. In a cosmetic composition for use on human skin from 0.1 to 10% by weight of a polymer as defined in any one of the preceding claims.